# EUROPEAN PATENT APPLICATION

(11) **EP 0 524 672 A1**
(43) Date of publication of application: **27.01.1993**
(21) Application number: 92201864.3
(22) Date of filing: 23.06.1992
(51) Int. Cl.: C12N 15/50, A61K 39/215, C12P 21/02, C07K 13/00

(54) **CCV vaccine**

(30) Priority: 27.06.1991 EP 91201640
(71) Applicant: DUPHAR INTERNATIONAL RESEARCH B.V, NL-1381 CP Weesp (NL)
(72) Inventor: Wesseling, J., NL-1380 AC Weesp (NL); Spaan, W., NL-1380 AC Weesp (NL); Rottier, P., NL-1380 AC Weesp (NL)
(74) Representative: Breepoel, Peter M.

(57) **Abstract**

The present invention is concerned with vaccine for combating CCV infection in canines containing proteins or polypeptides typical of the peplomer protein of CCV or containing recombinant polynucleotides having as part thereof a polynucleotide coding for said protein or polypeptide, and also is concerned with the preparation of said proteins, polypeptides and polynucleotides.

## Description

The present invention is concerned with a vaccine for combating Canine Corona Virus (CCV) infection and with recombinant polynucleotides and polypeptides for the preparation of such a vaccine.

The CCV belongs to the group of the so-called coronaviridae. The coronaviruses owe their name to the morphology of the virus particle. This particle is made up of an RNA molecule and three proteins, termed N, E₁ and E₂. The RNA molecule and the N protein (nucleocapsid protein) together constitute the core of the virus particle. This core is surrounded by a membrane, composed of E₁ and E₂ proteins. The E₂ protein appears in the form of clearly stalked protrusions (so-called spikes or peplomers), which give the virus particle the appearance of a crown (corona).

An infection of dogs with CCV may result in among others enteritis. Even though dogs of all ages are sensitive to CCV infection, young puppies are much more sensitive than older dogs. Clinical symptoms emerge within 1-5 days after infection. The symptoms vary from a decrease in appetite to fever, diarrhea, vomiting and lethargy. The very watery diarrhea may last for more than 10 days, implying a considerable risk of dehydration. Young puppies, in particular, run a considerable risk of dehydration and the lethality can be enormous. Secondary infections (by bacteria or by parvovirus) may increase the syndrome considerably.

Much research has been done to develop an adequate vaccine against CCV. This led to the understanding that protection is obtained only by local immunity in the intestines. Hence, vaccination needs to be aimed at induction of a local immune response in the intestines. The value of parenterally applied CCV vaccines is questionable.

An inactivated vaccine against CCV infections recently had to be withdrawn as it was shown to cause side effects such as encephalitis. Hence, there is a need to a safe and effective vaccine against CCV infections.

According to the present invention a vaccine can be provided based on a polynucleotide coding for the production of the E₂ protein or part thereof, or on a synthetic polypeptide corresponding to at least part of the E₂ protein.

The present invention is based on the discovery of the nucleotide sequence coding for the E₂ protein of CCV, and which is represented in Sequence ID No: 1. From this nucleotide sequence the amino acid sequence of the E₂ protein could be derived, which is represented in Sequence ID No: 1 too.

Accordingly, the present invention is concerned with E₂ protein and polypeptide E₂ protein fragments (E₂ polypeptides) having at least part of the amino acid sequence of Sequence ID NO: 1.

More in particular, the E₂ polypeptides of interest are able to elicit an immune response against CCV upon administration to dogs.

Hence, it goes without saying that the present invention is not only concerned with DNA of Sequence ID NO: 1 and fragments thereof, but also regards polynucleotides which hybridize to said DNA and fragments thereof and which codes for a polypeptide having the immunogenic properties of the E₂ protein of CCV.

The present invention is concerned also with a polynucleotide which codes for a polypeptide having the immunogenic properties of the E₂ protein of CCV wherein at least part of the codons of the DNA of Sequence ID NO:1 or of the fragments thereof, or of the abovementioned hybridizing polynucleotide is replaced by alternative codons for the same amino acid.

Small antigens often are useless as immunogens. Therefore the E₂ polypeptides may be prepared as homopolymers (a multitude of indentical E₂ polypeptides coupled) or heteropolymers (one or more E₂ polypeptide coupled to one or more different E₂ polypeptides, or coupled to one or more different polypeptides characteristic of CCV or an other pathogen), or may be coupled to one or more other compounds in order to enhance immunogenicity.

According to a particular embodiment of the present invention the E₂ polypeptide, in any of the modifications mentioned above, may be prepared synthetically, e.g. by homogeneous or by solid state polypeptide synthesis.

The particular amino acid sequences of suitable E₂ polypeptides may be derived from the amino acid sequence according to Sequence ID No:1 and optionally also from the spatial configuration of the E₂ protein. Suitable E₂ polypeptides may be selected from the most hydrophilic parts of the E₂ protein, e.g. by applying the technique described by Hopp and Woods. Another suitable method for selecting such polypeptides is described by Chou and Fasman.

A number of methods has been developed to predict the location of immunogenically important epitopes on proteins. The outcome of the combined predictions gives a good forecast of antigenic sites.

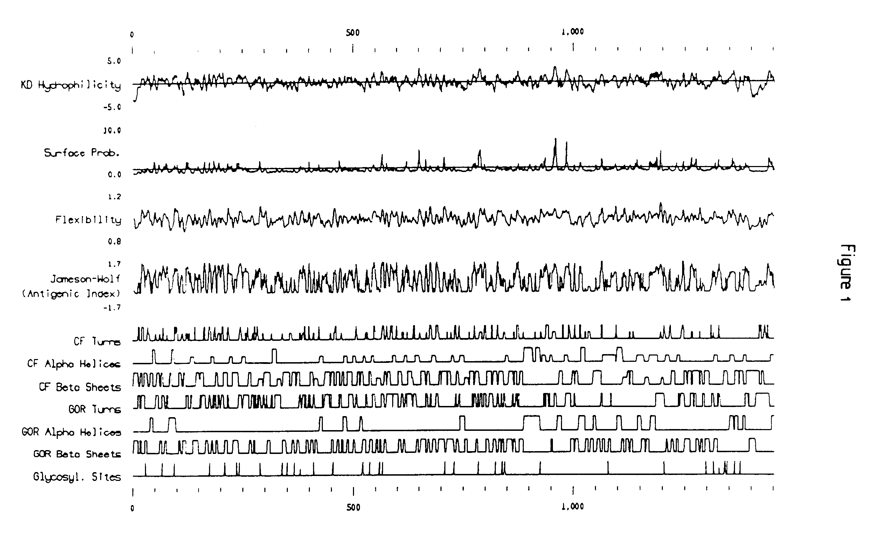

Figure 1 shows the various algorithms that have been used to finally predict the antigenically important regions at the CCV-E₂-protein.

The Hydrophilicity Plot was made according to Kyte and Doolittle [J. Mol. Biol., 157; 105-132 (1982)].

Surface Probability was determined using the formula of Emini et al. where the single probabilities were taken from Janin et al [J. Mol. Biol., 125; 357-386 (1978)].

The Chain Flexibility was estimated by using the method of Karplus et al [Naturwissenschaften, 72; 212-213 (1985)].

The Antigenicity Index (i.e. the probability that a region is antigenic) was calculated as described by Jameson and Wolf [CABIOS, 2 (1); 181-186 (1988)].

Turns, α-helices and β-sheets are predicted using the Chou and Fasman method [Chou P.Y. and Fasman G.D. (1987) Advances in Enzymology 47, 45-148] and the (slightly modified) method of Robson-Garnier [Garnier et al. (1987): J. Mol. Biol., 120; 97]. Additional information on the location of relevant epitopes can be obtained using the PEPSCAN-method, developed by Geysen and Meloen [Geysen, H.M., Meloen, R.H. and Barteling, S.J. (1984): Proc. Natl. Acad. Sci., 81 (13); 3998-4002].
This method uses synthetic peptides of sufficient length to react in an enzyme-linked immunosorbent assay. These peptides are synthesised according to a given DNA-sequence. They are characterised by the fact that the first peptide covers amino acid nr. 1-9, the second peptide covers amino acid nr. 2-10 etc.

Each peptide is tested for its reactivity with antisera or monoclonal antibodies. Reactive peptides must then represent an immunogenic epitope.

According to an other particular embodiment of the present invention an E₂-protein-specific polypeptide is produced by expression of a polynucleotide having at least part of the polynucleotide Sequence ID No:1 forming part of a recombinant polynucleotide. The recombinant polynucleotide preferably may be based on a vector with a CCV-specific polynucleotide fragment inserted therein. Suitable vectors are plasmids, bacteriophages, cosmids, viruses, minichromosomes or stably integrating vectors; the latter in particular for plant or animal cells. Generally these vectors have the property of atonomous replication except for the stably integrating vectors which insert themselves in the genetic material of the host cell and replicate with the host's genetic material. Suitable host cells may be either prokaryotic or eukaryotic, such as bacteria, yeasts, mycoplasms, algae, plant cells or animal cells; the plant cells or animal cells may be cultivated in vitro or may form part of an intact plant or animal, respectively. The recombinant polynucleotide may contain as an insert a complete polynucleotide coding for E₂ protein or a fragment thereof. The insert may comprise a single coding sequence, or `multiple copies of the same coding sequence, or a hybrid polynucleotide containing at least one E₂ protein coding sequence and at least one second sequence such as a different part of the E₂ protein coding sequence or a polynucleotide coding for a protein characteristic of an other pathogen or for an inert protein functioning as a carrier for a small E₂-polypeptide.

A specific case of the above embodiment is concerned with recombinant polynucleotides with viral vectors, directly useful as so-called vector vaccines. The viruses applicable for this purpose should have the ability to replicate in the animals to be immunized, i.e. in canines. These viruses, furthermore, should possess a genomic region suitable for insertion of a foreign gene (e.g. coding for the CCV-E₂ protein or polypeptide) which is also to be expressed in the vaccinated animal. Suitable viruses for this purpose are for example enteral viruses such as certain adeno viruses.

As was indicated above, the proteins and polypeptides, and the recombinant polynucleotides according to the invention are useful in the preparation of vaccines. Hence, these vaccines also form part of the invention.

A particular application of the present invention is concerned with bacterial vector vaccines. Herein bacteria capable of colonizing canines are transformed in order to enable them to express an E₂ protein or E₂ polypeptide in such a way that it will lead to an immunogenic respons against CCV. Suitable bacteria for this purpose are e.g. Salmonella bacteria.

A vaccine according to the invention may also contain auxiliary vaccine constituents, such as carriers buffers, stabilizers, solubilizers, adjuvants and preservatives. Advantageously these vaccines are freeze-dried products which are reconstituted by the addition of a suitable liquid (water, buffer) prior to application.

The vaccine can be applied orally, intranasally or intramuscularly.

The vaccine may additionally contain other immunogens for canines, such as immunogenic material characteristic of canine parvo virus, canine adeno virus. Bordetella bronchiseptica, Campilobacter jejuni, Campilobacter coli, Yersinia enterocolitica, Leptospira interrogans and Borrelia burgdorferi.

The invention is illustrated by the following working Examples.

### EXAMPLE 1.

### ISOLATION AND SEQUENCING OF CCV E₂ GENE

### Cells and virus

Feline catus whole foetus (fcwf) cells were grown in Dulbecco's modified Eagle's medium (DMEM) supplemented with 10% fetal calf serum (FCS), penicillin (100 IU) and streptomycin (100 microg/ml). The canine coronavirus (CCV) strain that was used (strain K378) was a field isolate, obtained from Duphar.

### Preparation of virus infected cells

CCV-K378 virus was plaque purified and grown on fcwf cells at 37°C. Confluent roller bottles (850 cm2; Corning) containing about 5 X 10⁷ cells were infected with high-titered CCV stocks with a multiplicity of infection of 0.1 TCID₅₀ in an inoculum of 20 ml phosphate buffered saline, containing 50 ug/ml DEAE-dextran (PBS-DEAE). Virus adsorption was continued for 1 h, the inoculum was removed and the cells were washed once with PBS. Subsequently, 25 ml of DMEM-10% FCS was added. 15 hours post-infection (beginning of cytopathic effect), the culture fluid was removed and the cells were washed with PBS.

### Isolation of RNA

For the isolation of RNA from virus infected cells, the cells were lysed with guanidinium thiocyanate (GSCN) as described by Maniatis et al. [Maniatis, T., Fritsch, E.F. and Sambrook, J. (1982): Molecular Cloning, A Laboratory Manual, Cold Spring Harbor Laboratory, New York]. The RNA was isolated by centrifugation through a cushion of 5.7 M CsCl, essentially as described by Maniatis et al. (1982). The RNA was further purified by phenol extractions and ethanol precipitations. Poly(A)⁺ RNA was isolated on an oligo (dT) cellulose column (Pharmacia) as described by Maniatis et al. (1982).

### cDNA synthesis

For cDNA synthesis the RNA was primed with calf thymus DNA pentamers and with oligo(dT) primers (both obtained from Pharmacia). First and second strand synthesis was carried out as described by Gubler and Hoffman [Gubler, U. and Hoffman, B.J. (1983): A simple and very efficient method for generating cDNA libraries. Gene 25, 263-269]. The cDNA was tailed with dC residues, annealed to a dG-tailed, PstI digested pUC9 plasmid (Pharmacia) an used for transformation of E.coli strain PC2495, as described by Hanahan [Hanahan, D. (1983): Studies on transformation of Escherichia coli with plasmids. J. Mol. Biol. 166, 557-580]. The cells were plated on petri dishes containing 50 µg/ml ampicilline. About 5000 independent transformants were obtained, which were screened for the presence of recombinants containing CCV-E₂ sequences by in situ colony hybridization with the ³²P-labelled S gene of FIPV [de Groot, R.J., Maduro, J., Lenstra, J.A., Horzinek, M.C., Van de Zeijst, B.A.M. and Spaan, W.J.M. (1987): cDNA cloning and sequence analysis of the gene encoding the peplomer protein of feline infectious peritonitis virus. J. Gen. Virol. 68, 2639-2646] as a heterologous probe.

### Isolation of CCV-E₂ cDNA clones

Hybridization of the CCV-cDNA library with the FIPV-S gene was carried out as described by Maniatis et al. (1982) DNA probes were prepared by nick-translation according to Maniatis et al. (1982). About 25 positive recombinant plasmids were obtained, which contained inserts ranging from 0.4 and 3.0 kb (mean length 1.5 kb). Small scale and large scale plasmid DNA isolations were prepared according to Maniatis et al. (1982) Restriction maps and physical maps of the CCV-cDNA clones were constructed by restriction enzyme analysis and by hybridization with different fragments of the FIPV-S gene.

### Sequencing of the CCV-E₂ gene

Restriction fragments of CCV-E₂ cDNA were separated by agarose gel electrophoresis and the DNA was purified from the agarose by glass-milk (GeneClean™). Subsequently, the DNA fragments were recloned into bacteriophage M13 mp18/mp19 vectors [Yanisch-Perron, C., Vieira, J. and Messing, J. (1985): Improved M13 phage cloning vectors and host strains: nucleotide sequences of the M13mp18 and pUC19 vectors. Gene 33, 103-119]. Sequencing was carried out according to the dideoxy-nucleotide chain-termination procedure of Sanger et al. [Sanger, F., Nicklen, S. and Coulson, A.R. (1977): DNA sequencing with chain terminating inhibitors. Proc. Natl. Acad. Sci, USA 74, 5463-5467].

DNA sequences encoding the CCV-E₂ gene were analyzed on a DEC computer, using the computer programs of [Staden, R. (1982): Automation of the computer handling of gel reading data produced by the shotgun method of DNA sequencing. Nucleic Acid Res, 10, 4731-4751] and the programs developed by the University of Wisconsin. The results are shown as Sequence ID No: 1.

### EXAMPLE 2.

### EXPRESSION OF THE CCV-E₂ GENE IN A VACCINIA-T₇ SYSTEM

### Materials and methods.

### Construction of the expression vector.

For expressing of the CCV-E₂ gene, the T₇ polymerase/promoter system as described by Fürst et al. [Fürst, T.R., Niles, E.G., Studier, F.N. and Moss, B. (1986) PNAS USA 83, 8122-8126] was used.
Basically, this is a two-component system, containing:
1) a plasmid containing the bacteriophage T₇ promoter and terminator with the gene of choice cloned in between.
2) a recombinant vaccinia virus recombinant containing the bacteriophage T₇ polymerase gene under the control of the P7.5 vaccinia promoter.

Transfection of suitable cells with the recombinant plasmid, followed by infection with the recombinant vaccinia virus then leads to expression of the foreign gene.

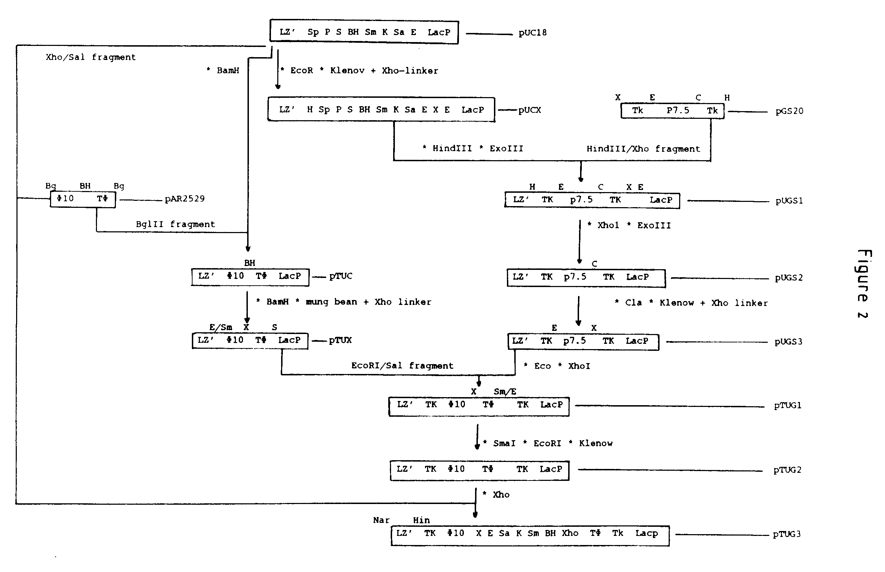

The expression-vector pTUG3 was constructed as described below and is schematically represented in Figure 2.

List of abbreviations used in Figure 2:
- Φ10:: T7 promoter
- TΦ:: T7 terminator
- LZ':: LacZ fragment of pUC18
- LacP:: Lac promoter
- TK:: thymidine kinase sequences
- P7.5:: vaccinia virus P7.5 promoter
- BH:: BamH1
- C:: Cla1
- E:: EcoR1
- H:: HinDIII
- K:: Knp1
- N:: Nar1
- P:: Pst1
- S:: Sac1
- S:: Sal1
- Sm:: Sma1
- Sp:: Sph1
- Xb:: Xba1
- X:: Xho1

The T7 promoter/terminator cassette from pAR2529 (Fürst et al, 1986) was cloned as a BglII fragment into the BamH1 site of pUC18. A clone with the T7 promoter in the opposite orientation to the lacZ promoter was named pTUC. After cleavage of pTUC with BamH1 the protruding 5'-ends of the BamH1 site were degraded with mung bean nuclease (in this process 8 additional nucleotides towards the T7 promoter were removed), and a linker containing an XhoI site (CCTCGAGG) was cloned between them, resulting in pTUX. In pUC18 a XhoI site was created by cloning the XhoI linker into an EcoR1 site filled in with Klenov polymerase, the resulting plasmid was called pUCX. Between the HindIII and XhoI site of this plasmid a 1.7kb HindIII/XhoI fragment of pGS20 (Mackett et al, 1984) was cloned. This fragment contained vaccinia TK sequences and the vaccinia P7.5 promoter. The resulting construct was named pUGS1. About 30 nucleotides of both sides of the XhoI site were deleted with exonuclease III. This resulted in pUGS2 which lacks the XhoI site and one of the two EcoR1 sites. In pUGS3 the ClaI site just upstream of the P7.5 promoter site was converted to an XhoI site by cloning an XhoI linker into the Klenov filled in ClaI site. For the construction of pTUG1 the P7.5 promoter XhoI/EcoR1 fragment of pUGS3 was replaced by the SalI/EcoR1 T7 promoter/terminator cassette of pTUX. Subsequently the SmaI/EcoR1 fragment was removed and the protruding ends were filled with Klenov polymerase, yielding pTUG2.

pTUG3 was finally constructed by cloning the XhoI/Sa1I polylinker fragment of pTUCX into the XhoI site of pTUG2. pTUG31 is a derivative of pTUG3 which lacks the 170 nucleotides long NarI/HindIII lacZ fragment of pUC18. The protruding ends were filled in with Klenov enzyme before ligation.

The final constructs contain T7 promoter and terminator sequences separated by a multiple cloning site and surrounded by vaccinia virus thymidine kinase sequences. The T7 promoter precedes a sequence that is capable of forming a hairpin structure in the transcript.

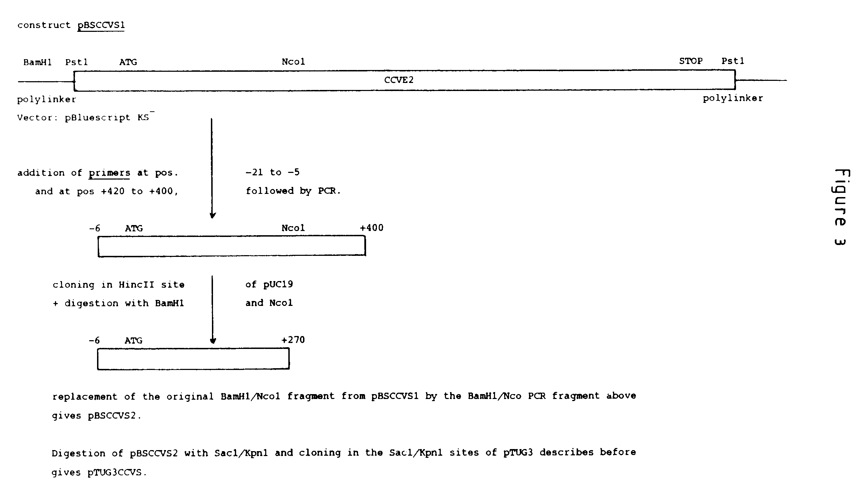

Starting from pTUG3, pTUG3CCVS was constructed as described below (see also Figure 3):
The 5'- and 3'-noncoding sequences of a cDNA Pst1 cassette containing the CCV-E₂ gene were removed from construct pBSCCVS1 (which was deposited at the Centraalbureau voor Schimmelcultures at Baarn, the Netherlands) as indicated in Figure 3.

List of abbreviations in Figure 3:
- CCVE2:: cDNA fragment containing the coding region of the CCV-spike gene flanked by 3'- and 5'-noncoding sequences, as a Pst1 cassette
- ATG:: translation-initiation codon of the CCVE2-gene,
- STOP:: the stop-codon of the same gene
- PCR:: polymerase chain reaction

Construct pBSCCVS1 contains the cDNA containing the CCV-E2 gene and flanking sequences as a Pst1 cassette. Addition of primers at position -21 to -6 and +420 to +400 followed by Polymerase Chain Reaction yielded a blunt-ended fragment that was subsequently cloned in the HincII site of pUC19. Digestion with BamHI and Nco1 gave a fragment that is almost free of 5'-untranslated nucleotides.

The BamHI/Nco1 fragment in the original pBSCCVS1 construct was subsequently cut out and replaced by the shortened BamHI/Nco1 fragment described above, yielding pBSCCVS2.

Finally, pBSCCVS2 and pTUG3 were digested with Sac1 and Kpn1. Cloning of the Sac1/Kpn1 fragment of pBSCCVS2 in the Sac1/Kpn1 site of pTUG3 gave pTUG2CCVS.

Thus pTUG3CCVS contains the coding region of the CCV spike-gene under the control of the T7 promoter and followed by two adjacent T7 terminators.

### Transient expression conditions.

Hela cells were grown to 80% confluency in 25 ml flasks and infected with recombinant vaccinia-T7 virus at a multiplicity of infection of 30 virus particles per cell.
The virus was allowed to absorb for 30 minutes at 37°C. A calcium phosphate precipitate was made, and 1 ml containing 15 µg DNA was added to the cells after removal of the virus-suspension. After 30 minutes incubation at room temperature, fresh medium containing 10% FCS was added to the cells, and the cells were kept at 37°C. At various times after transfection, cells were harvested.

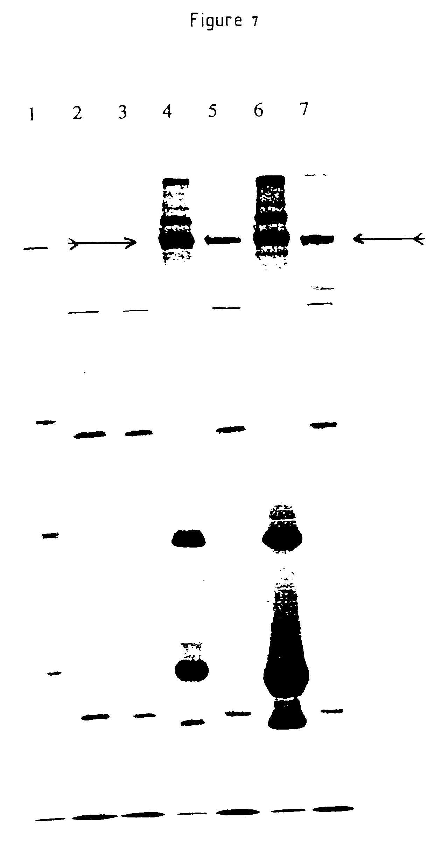

Figure 7 shows the expression of the CCV-E2 protein under the control of the T7 promoter. The E2-protein is indicated by an arrow.

³⁵S-labeled radio-immune precipitates were used on a 10% poly-acrylamide gel.

### Lane

- 1:: ¹⁴C-marker,
- 2:: vaccinia-virus infected cells,
- 3:: vaccinia-inf. + pTUG3,
- 4:: CCV-infected cells,
- 5:: vaccinia-inf. + pTUG3CCVS,
- 6:: FIPV-infected cells,
- 7:: vaccinia-inf. + pTUG3FIPV-S.

Note: FIPV and FIPV-constructs were used as a marker.

### EXAMPLE 3.

### CLONING AND RESCUE OF THE CCV-E₂ GENE INTO ADENOVIRUS Ad5

### MATERIALS

### Cells, viruses and plasmids

Ad5-transformed human embryo kidney cells (293 cells) were used for rescue of viruses and were obtained from F.L. Graham (McMaster University) [Graham, F.L., Smiley, J., Russel, W.C. and Nairn, R. (1977): Characteristics of a human cell line transformed by DNA from human adenovirus 5 DNA. J. Gen. Virol 36, 59-72]. For titration and growth of viruses also HeLa cells were used. Monolayer cultures of cells were maintained in MEM F-11 medium. Media were supplemented with 5% Foetal Calf Serum. All cell culture was carried out in incubators at 37°C, 100% humidity, and an atmosphere of 5% C0₂. Ad5 wild-type virus and Ad5 recombinant viruses were obtained and rescued in cooperation with F.L. Graham (McMaster University).

All enzymes used for recombinant DNA work were purchased from Pharmacia, Gibco-BRL and Boehringer-Mannheim and used under reaction conditions recommended by the vendor. Plasmid DNA was prepared using the alkaline lysis method [Birnboim, H.C. and Doly, J. (1979): A rapid alkaline extraction procedure for screening recombinant plasmid DNA. Nucleic Acids Res. 7, 1513-1523] and the DNA was further purified on CsCl-ethidium bromide density gradients. DNA fragments were eluted and purified from agarose using the GeneCleane procedure.

### METHODS

### Cloning of the CCV-E₂ gene in Ad5

### 1. Construction of pSV-plasmids

Plasmid pSV2X13 has been derived from pSV2X11 (F.L. Graham, unpublished); this latter plasmid has been derived from pSV2X13 (L. Prevec, unpublished). Plasmid pSV2X3 was derived from pSV2neo [Southern, P.J. and Berg, P. (1982):
Transformation of mammalian cells to antibiotic resistance with a bacterial gene under control of the SV40 early region promoter. J. Mol Appl. Genet. 1, 327-334]. pSV2X3 has the SV40 promoter (SV49 prom.) sequence and poly(A) addition sequence ((A)ₙ) separated by a polylinker sequence that is used to insert appropriate genes. The cassette is bounded by XbaI sites. To construct pSV2X11 a deletion has been made between the polylinker and the SV40 promoter of pSV2X3 and a unique XhoI site has been created at this position.

Subsequently, the ampicillin resistance gene (Ampᵣ)has been removed from its original position, XhoI linkers were added and the gene was subcloned into the unique XhoI site of pSV2X3. The resultant pSV2X11 plasmid now has the ampicillin resistance gene flanked by two XhoI sites. pSV2X13 was derived from pSV2X11 by inactivation of one of both EcoRI sites, in order to have a unique EcoRI site in the polylinker.

### 2. Construction of adenovirus transfer vectors.

Plasmid pAB14 was derived from transfervector pFG144K3 [F.L. Graham, unpublished]. pFG144K3 consists of the rightward 40% of Ad5 DNA fused to a plasmid containing an origin of replication and a kanamycin resistance gene (Km^{r}). The Ad5 part of pFG144K3 has a deletion in the essential El region (1.3-10.6 map units) and a deletion in the non-essentiel E3 region (78.5-84.7 m.u.). In pAB14 the deletion in the E3 region has been increased (78.3-85.0 m.u.), in order to clone larger genes in Ad5. Also in pAB14, the XhoI and SalI sites in the Ad5 part of pFGI44K3 also have been inactivated. Plasmid pAB16 was derived from pAB14 [F.L. Graham, unpublished] by insertion of a polylinker in the unique XbaI site of pAB14. In pAB16 genes can be cloned directly into the deleted E3 region of Ad5 without being flanked by the SV40 promoter and SV40 poly(A) addition sequences. When the gene of interest is cloned in a suitable transfer vector, this construct together with a second plasmid pFG173 containing complementing Ad sequences were cotransfected into 293 cells (Graham et al., 1977). pFG173 can complement sequences from the left end of Ad5 necessary for virus replication and carries a lethal deletion comprising the region between 75.9 and 85.0 m.u. of the Ad genome. Each of the plasmids alone (adenotransfer vector and pFG173) are not infectious.

### 3. Cloning of the CCV-E₂ gene in adenovirus transfer vectors

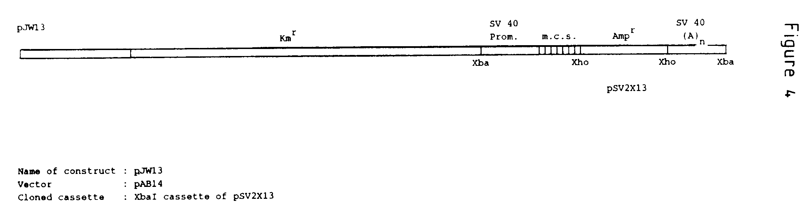

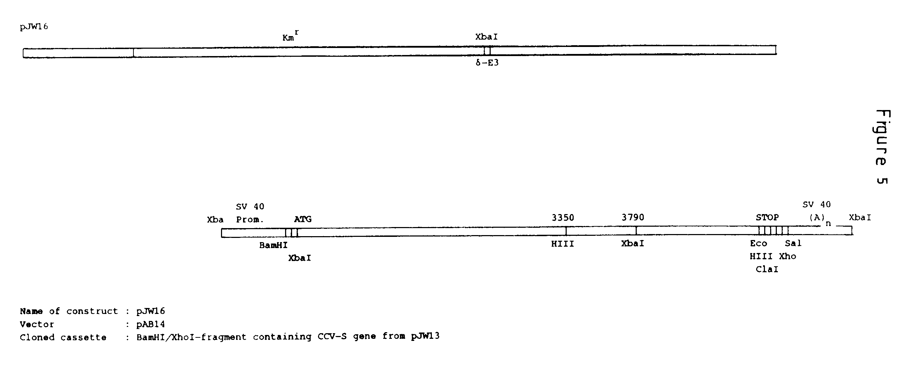

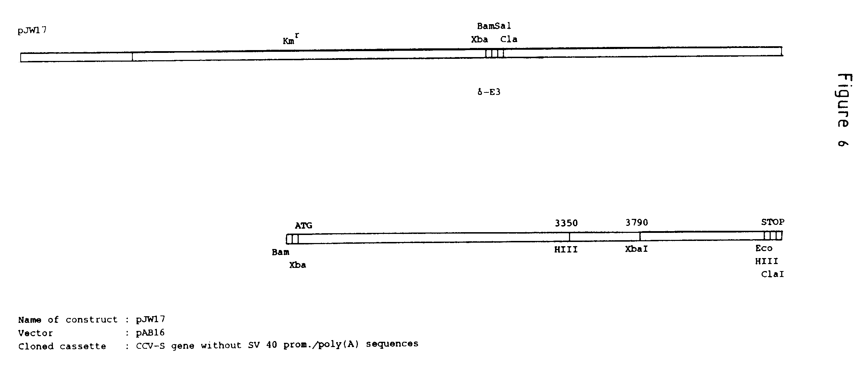

The CCV-E₂ gene has been cloned into the Pstl and EcoRl sites of the polylinker of pBLueScript KS-. This plasmid was derived from pUC19 (Yanisch-Perron et al., 1985). Subsequently, the 5' non-coding region and the dG/dC-tail at the 5' end of the CCV-E₂ gene were removed. The CCV-S gene is subsequently cloned into adenovirus transfer vector pJW13 (Figure 4). This transfer vector is constructed by cloning the XbaI cassette of pSV2Xl3 (containing the SV40 promoter, polylinker, ampicillin resistance gene and the SV40 polyadenylation signal sequences) into XbaI site of pABl4. pJW13 contains both ampicillin and kanamycin resistance genes. The orientation of the SV40 promoter in pJW13 is parallel with respect to the E3 promoter. The CCV-E₂ gene is cloned into the BamHl and Xhol(Xho) sites of pJW13 resulting in the loss of the ampicillin gene. This construct is called pJW16 (Figure 5), and the CCV-E₂ gene is expressed using the SV40-promoter/poly(A) signal sequences. The CCV-E₂ gene is also cloned directly (i.e. without the presence of the SV40 promoter/SV40 polyadenylation signal sequences) into pAB16 using the BamHl and Sall sites of the deleted E3 region. The CCV-E₂ gene was cloned in such a way that the orientation is parallel to the direction of transcription of the adenovirus E3 promoter. This construct is designated pJW17 (Figure 6).

### Rescue and purification of recombinant M5 viruses

Semiconfluent monolayers of 293 cells in 60-mm dishes were cotransfected with plasmid pFG173 plus pJW16/pJW17 plasmids by the calcium phosphate precipitation technique [Graham, F.L. and Van der Eb, A.J. (1973): A new technique for assay of infectivity of adenovirus 5 DNA. Virology 52, 456-467]. In this way recombinant Ad5 viruses containing the CCV-E₂ gene were rescued. The Ad5 viruses are called Ad5CCV-Sl (pJW16 derivative) and Ad5CCV-S2 (pJW17 derivative). After 8-10 days individual plaques were isolated and used to infect 60 mm dishes of 293 cells. Viral DNA was analyzed by digestion with HindIII or XbaI and electrophoresis in 1% agarose gels. Recombinant Ad viruses were plaque purified twice and reanalyzed by restriction enzyme digestions. Viruses were grown in HeLa cells to obtain large scale preparation of virus stocks.

### Rescue and purification of recombinant Ad5 viruses

Semiconfluent momonolayers of 293 cells in 60-mm dishes were cotransfected with plasmid pFG173 plus pJW16/pJW17 plasmids (see Figure 4,5 and 6) by the calcium phosphate precipitation technique (Graham et al., 1973). In this way recombinant Ad5 viruses containing the CCV-E₂ gene were rescued. The Ad5 viruses are called Ad5CCV-S1 (pJW16 derivative) and Ad5CCV-S2 (pJW17 derivative). After 8-10 days individual plaques were isolated and used to infect 60 mm dishes of 293 cells. Viral DNA was analyzed by digestion with HindIII or XbaI and electrophoresis in 1% agarose gels. Recombinant Ad viruses were plaque purified twice and reanalyzed by restriction enzyme digestions. Viruses were grown in HeLa cells to obtain large scale preparation of virus stocks.

SEQUENCE LISTING

INFORMATION FOR SEQUENCE ID NO: 1

SEQUENCE CHARACTERISTICS:
(A) LENGTH: 4462 base pairs
(B) TYPE: nucleic acid
(C) STRANDEDNESS: double
(D) TOPOLOGY: linear
MOLECULE TYPE: cDNA to genomic RNA
ORIGINAL SOURCE:
(A) ORGANISM: Canine Corona Virus
(B) STRAIN: K378
IMMEDIATE EXPERIMENTAL SOURCE
NAME OF CELL LINE: CBS 485.90
FEATURE:
(A) LOCATION: from 169 to 4455 base pairs
(B) OTHER INFORMATION: open reading frame
PROPERTIES: Spike protein (= E2 protein)

SEQUENCE DISCRIPTION: SEQ ID NO:

TGTTAATTTA AAAGAAAAAG AATTGAATGA AATGGTTGTT GGATTACTAA GGAAAGGTAA 60
GTTGCTCATT AGAAACAATG CAAAACTACT AAACTTTGGT AATCACTTGG TTAATGTGCC 120
ATG ATT GTG CTA ATA TTG TGC CTC CTC TTG TTT TCG TAC AAT AGT GTG 168
Met Ile Val Leu Ile Leu Cys Leu Leu Leu Phe Ser Tyr Asn Ser Val
1               5                   10                  15

ATT TGT ACA TCA AAC AAT GAC TGT GTA CAA GGT AAT GTG ACA CAA TTG 216
Ile Cys Thr Ser Asn Asn Asp Cys Val Gln Gly Asn Val Thr Gln Leu
            20                  25                  30

CCT GGC AAT GAA AAC ATT ATT AAA GAT TTT CTA TTT CAC ACC TTC AAA 264
Pro Gly Asn Glu Asn Ile Ile Lys Asp Phe Leu Phe His Thr Phe Lys
        35                  40                  45

GAA GAA CCA AGT GTA GTT GTT GGT GGT TAT TAC CCT ACA GAG GTG TGG 312
Glu Glu Pro Ser Val Val Val Gly Gly Tyr Tyr Pro Thr Glu Val Trp
    50                  55                  60

TAT AAC TGC TCC AGA AGC GCA ACA ACC ACC GCT TAC AAG GAT TTT AGT 360
Tyr Asn Cys Ser Arg Ser Ala Thr Thr Thr Ala Tyr Lys Asp Phe Ser
65                  70                  75                  80

AAT ATA CAT GCA TTC TAT TTT GAT ATG GAA GCC ATG GAG AAT AGT ACT 408
Asn Ile His Ala Phe Tyr Phe Asp Met Glu Ala Met Glu Asn Ser Thr
                85                  90                  95

GGC AAT GCA CGA GGT AAA CCT TTA CTA GTA CAT GTT CAT GGT GAT CCT 456
Gly Asn Ala Arg Gly Lys Pro Leu Leu Val His Val His Gly Asp Pro
            100                 105                 110

GTT AGT ATC ATC ATA TAT ATA TCG GCT TAT AGA GAT GAT GTG CAA CCA 504
Val Ser Ile Ile Ile Tyr Ile Ser Ala Tyr Arg Asp Asp Val Gln Pro
        115                 120                 125

AGG CCT CTT TTA AAA CAT GGT TTG TTG TGT ATA ACT AAA AAT AAA ATC 552
Arg Pro Leu Leu Lys His Gly Leu Leu Cys Ile Thr Lys Asn Lys Ile
    130                 135                 140

ATT GAC TAT AAC ACG TTT ACC AGC GCA CAG TGG AGT GCC ATA TGT TTG 600
Ile Asp Tyr Asn Thr Phe Thr Ser Ala Gln Trp Ser Ala Ile Cys Leu
145                 150                 155                 160

GGT GAT GAC AGA AAA ATA CCA TTC TCT GTC ATA CCC ACA GAT AAT GGT 648
Gly Asp Asp Arg Lys Ile Pro Phe Ser Val Ile Pro Thr Asp Asn Gly
                165                 170                 175

ACA AAA ATA TTT GGT CTT GAG TGG AAT GAT GAC TAT GTT ACA GCC TAT 696
Thr Lys Ile Phe Gly Leu Glu Trp Asn Asp Asp Tyr Val Thr Ala Tyr
            180                 185                 190

ATT AGT GAT CGT TCT CAC CAT TTG AAC ATC AAT AAT AAT TGG TTT AAC 744
Ile Ser Asp Arg Ser His His Leu Asn Ile Asn Asn Asn Trp Phe Asn
        195                 200                 205

AAT GTG ACA ATC CTA TAC TCT CGA TCA AGC TCT GCT ACG TGG CAG AAG 792
Asn Val Thr Ile Leu Tyr Ser Arg Ser Ser Ser Ala Thr Trp Gln Lys
    210                 215                 220

AGT GCT GCA TAT GTT TAT CAA GGT GTT TCA AAT TTT ACT TAT TAC AAG 840
Ser Ala Ala Tyr Val Tyr Gln Gly Val Ser Asn Phe Thr Tyr Tyr Lys
225                 230                 235                 240

TTA AAT AAC ACC AAT GGC TTG AAA AGC TAT GAA TTG TGT GAA GAT TAT 888
Leu Asn Asn Thr Asn Gly Leu Lys Ser Tyr Glu Leu Cys Glu Asp Tyr
                245                 250                 255

GAA TAC TGC ACT GGC TAT GCT ACC AAC GTA TTT GCC CCG ACA GTG GGC 936
Glu Tyr Cys Thr Gly Tyr Ala Thr Asn Val Phe Ala Pro Thr Val Gly
            260                 265                 270

GGT TAT ATA CCT CAT GGC TTC AGT TTT AAC AAT TGG TTT ATG CGG ACA 984
Gly Tyr Ile Pro His Gly Phe Ser Phe Asn Asn Trp Phe Met Arg Thr
        275                 280                 285

AAC AGT TCC ACG TTT GTT AGT GGC AGA TTT GTA ACA AAT CAA CCA TTA 1032
Asn Ser Ser Thr Phe Val Ser Gly Arg Phe Val Thr Asn Gln Pro Leu
    290                 295                 300

TTG GTT AAT TGT TTG TGG CCA GTG CCC AGT TTT GGT GTC GCA GCA CAA 1080
Leu Val Asn Cys Leu Trp Pro Val Pro Ser Phe Gly Val Ala Ala Gln
305                 310                 315                 320

CAA TTT TGT TTT GAA GGT GCG CAG TTT AGC CAA TGT AAT GGT GTG TCT 1128
Gln Phe Cys Phe Glu Gly Ala Gln Phe Ser Gln Cys Asn Gly Val Ser
                325                 330                 335

TTA AAC AAT ACA GTG GAT GTC ATT AGA TTC AAC CTT AAT TTT ACC GCA 1176
Leu Asn Asn Thr Val Asp Val Ile Arg Phe Asn Leu Asn Phe Thr Ala
            340                 345                 350

TTA GTA CAA TCT GGC ATG GGT GCT ACA GTA TTT TCA CTG AAT ACA ACA 1224
Leu Val Gln Ser Gly Met Gly Ala Thr Val Phe Ser Leu Asn Thr Thr
        355                 360                 365

GGT GGT GTC ATT CTT GAG ATT TCT TGT TAT AAT GAT ACA GTG AGT GAG 1272
Gly Gly Val Ile Leu Glu Ile Ser Cys Tyr Asp Asp Thr Val Ser Glu
    370                 375                 380

TCA AGT TTC TAC AGT TAT GGT GAA ATT TCA TTC GGC GTA ACT GAT GGA 1320
Ser Ser Phe Tyr Ser Tyr Gly Glu Ile Ser Phe Gly Val Thr Asp Gly
385                 390                 395                 400

CCG CGT TAC TGT TTC GCA CTC TAT AAT GGC ACG GCT CTT AAG TAT TTA 1368
Pro Arg Tyr Cys Phe Ala Leu Tyr Asn Gly Thr Ala Leu Lys Tyr Leu
                405                 410                 415

GGA ACA TTA CCA CCT AGT GTC AAG GAA ATT GCT ATT AGT AAG TGG GGC 1416
Gly Thr Leu Pro Pro Ser Val Lys Glu Ile Ala Ile Ser Lys Trp Gly
            420                 425                 430

CAT TTT TAT ATT AAT GGT TAC AAT TTC TTT AGC ACT TTT CCT ATT GAT 1464
His Phe Tyr Ile Asn Gly Tyr Asn Phe Phe Ser Thr Phe Pro Ile Asp
        435                 440                 445

TGT ATA TCT TTT AAT TTA ACC ACT GGT GAT AGT GGA GCA TTT TGG ACA 1512
Cys Ile Ser Phe Asn Leu Thr Thr Gly Asp Ser Gly Ala Phe Trp Thr
    450                 455                 460

ATT GCT TAC ACA TCG TAC ACT GAC GCA TTA GTA CAA GTT GAA AAC ACA 1560
Ile Ala Tyr Thr Ser Tyr Thr Asp Ala Leu Val Gln Val Glu Asn Thr
465                 470                 475                 480

GCT ATT AAA AAG GTG ACG TAT TGT AAC AGT CAC ATT AAT AAC ATT AAA 1608
Ala Ile Lys Lys Val Thr Tyr Cys Asn Ser His Ile Asn Asn Ile Lys
                485                 490                 495

TGT TCT CAA CTT ACT GCT AAT TTG CAA AAT GGA TTT TAT CCT GTT GCT 1656
Cys Ser Gln Leu Thr Ala Asn Leu Gln Asn Gly Phe Tyr Pro Val Ala
            500                 505                 510

TCA AGT GAA GTT GGT CTT GTC AAT AAG AGT GTT GTG TTA CTA CCT AGT 1704
Ser Ser Glu Val Gly Leu Val Asn Lys Ser Val Val Leu Leu Pro Ser
        515                 520                 525

TTC TAT TCA CAT ACC AGT GTT AAT ATA ACT ATT GAT CTT GGT ATG AAG 1752
Phe Tyr Ser His Thr Ser Val Asn Ile Thr Ile Asp Leu Gly Met Lys
    530                 535                 540

CGT AGT GGT TAT GGT CAA CCC ATA GCC TCA ACA TTA AGT AAC ATC ACA 1800
Arg Ser Gly Tyr Gly Gln Pro Ile Ala Ser Thr Leu Ser Asn Ile Thr
545                 550                 555                 560

CTA CCA ATG CAG GAT AAT AAC ACC GAT GTG TAC TGC ATT CGT TCT AAC 1848
Leu Pro Met Gln Asp Asn Asn Thr Asp Val Tyr Cys Ile Arg Ser Asn
                565                 570                 575

CGA TTT TCA GTT TAC TTT CAT TCC ACT TGT AAA AGT TCT TTA TGG GAC 1896
Arg Phe Ser Val Tyr Phe His Ser Thr Cys Lys Ser Ser Leu Trp Asp
            580                 585                 590

GAT GTG TTT AAT TCC GAC TGC ACA GAT GTT TTA TAT GCT ACA GCT GTT 1944
Asp Val Phe Asn Ser Asp Cys Thr Asp Val Leu Tyr Ala Thr Ala Val
        595                 600                 605

ATA AAA ACT GGC ACT TGT CCT TTC TCG TTT GAT AAA TTG AAC AAT TAC 1992
Ile Lys Thr Gly Thr Cys Pro Phe Ser Phe Asp Lys Leu Asn Asn Tyr
    610                 615                 620

TTA ACT TTT AAC AAG TTC TGT TTG TCA TTG AAT CCT GTT GGT GCC AAC 2040
Leu Thr Phe Asn Lys Phe Cys Leu Ser Leu Asn Pro Val Gly Ala Asn
625                 630                 635                 640

TGC AAG TTT GAT GTT GCC GCT CGT ACA AGA ACC AAT GAG CAG GTT GTT 2088
Cys Lys Phe Asp Val Ala Ala Arg Thr Arg Thr Asn Glu Gln Val Val
                645                 650                 655

AGA AGT TTA TAT GTA ATA TAT GAA GAA GGA GAC AAC ATA GTG GGT GTG 2136
Arg Ser Leu Tyr Val Ile Tyr Glu Glu Gly Asp asn Ile Val Gly Val
            660                 665                 670

CCG TCT GAC AAT AGT GGT CTT CAC GAC TTG TCA GTG CTA CAC TTA GAC 2184
Pro Ser Asp Asn Ser Gly Leu His Asp Leu Ser Val Leu His Leu Asp
        675                 680                 685

TCC TGT ACA GAT TAT AAT ATA TAT GGT ATA ACT GGT GTT GGT ATT ATT 2232
Ser Cys Thr Asp Tyr Asn Ile Tyr Gly Ile Thr Gly Val Gly Ile Ile
    690                 695                 700

AGA CAA ACT AAC AGT ACG CTA CTT AGT GGC TTA TAT TAC ACA TCA CTA 2280
Arg Gln Thr Asn Ser Thr Leu Leu Ser Gly Leu Tyr Tyr Thr Ser Leu
705                 710                 715                 720

TCA GGT GAC TTG TTA GGG TTT AAA AAT GTT AGT GAT GGT GTC ATC TAT 2328
Ser Gly Asp Leu Leu Gly Phe Lys Asn Val Ser Asp Gly Val Ile Tyr
                725                 730                 735

TCT GTC ACG CCA TGT GAT GTA AGC GCA CAT GCT GCT GTT ATT GAT GGC 2376
Ser Val Thr Pro Cys Asp Val Ser Ala His Ala Ala Val Ile Asp Gly
            740                 745                 750

GCC ATA GTT GGA GCT ATG ACT TCC ATT AAT AGT GAA CTG TTA GGT CTA 2424
Ala Ile Val Gly Ala Met Thr Ser Ile Asn Ser Glu Leu Leu Gly Leu
        755                 760                 765

ACA CAT TGG ACA ACA ACA CCT AAT TTT TAT TAT TAT TCT ATA TAT AAT 2448
Thr His Trp Thr Thr Thr Pro Asn Phe Tyr Tyr Tyr Ser Ile Tyr Asn
    770                 775                 780

TAT ACC AAT GAA AGG ACT CGT GGC ACA GCA ATT GAT AGT AAC GAT GTT 2496
Tyr Thr Asn Glu Arg Thr Arg Gly Thr Ala Ile Asp Ser Asn Asp Val
785                 790                 795                 800

GAT TGT GAA CCT ATC ATA ACC TAT TCT AAT ATA GGT GTT TGT AAA AAT 2544
Asp Cys Glu Pro Ile Ile Thr Tyr Ser Asn Ile Gly Val Cys Lys Asn
                805                 810                 815

GGA GCT TTG GTT TTT ATT AAC GTC ACA CAT TCT GAT GGA GAC GTT CAA 2592
Gly Ala Leu Val Phe Ile Asn Val Thr His Ser Asp Gly Asp Val Gln
            820                 825                 830

CCA ATT AGC ACC GGT AAT GTC ACG ATA CCT ACA AAT TTT ACC ATA TCT 2640
Pro Ile Ser Thr Gly Asn Val Thr Ile Pro Thr Asn Phe Thr Ile Ser
        835                 840                 845

GTG CAA GTT GAA TAC ATT CAG GTT TAC ACT ACA CCG GTG TCA ATA GAT 2688
Val Gln Val Glu Tyr Ile Gln Val Tyr Thr Thr Pro Val Ser Ile Asp
    850                 855                 860

TGT TCA AGG TAC GTT TGC AAT GGT AAC CCT AGA TGC AAT AAA TTG TTA 2736
Cys Ser Arg Tyr Val Cys Asn Gly Asn Pro Arg Cys Asn Lys Leu Leu
865                 870                 875                 880

ACG CAA TAC GTT TCT GCA TGT CAA ACT ATT GAG CAA GCA CTT GCA ATG 2784
Thr Gln Tyr Val Ser Ala Cys Gln Thr Ile Glu Ala Leu Ala Met Gly
                885                 890                 895

GGT GCC AGA CTT GAA AAC ATG GAG ATT GAT TCC ATG TTG TTT GTT TCG 2832
Gly Ala Arg Leu Gln Asn Met Glu Ile Asp Ser Met Leu Phe Val Ser
            900                 905                 910

GAA AAT GCC CTT AAA TTG GCA TCT GTT GAA GCA TTC AAT AGT ACG GAA 2880
Glu Asn Ala Leu Lys Leu Ala Ser Val Glu Ala Phe Asn Ser Thr Glu
        915                 920                 925

ACT TTA GAT CCT ATT TAC AAA GAA TGG CCT AAC ATT GGT GGT TCT TGG 2928
Thr Leu Asp Pro Ile Tyr Lys Glu Trp Pro Asn Ile Gly Gly Ser Trp
    930                 935                 940

CTA GGA GGT TTA AAA GAC ATA TTG CCA TCT CAC AAC AGC AAA CGT AAG 2976
Leu Gly Gly Leu Lys Asp Ile Leu Pro Ser His Asn Ser Lys Arg Lys
945                 950                 955                 960

TAC CGG TCG GCT ATA GAA GAT TTG CTT TTT GAT AAG GTT GTA ACA TCT 3024
Tyr Arg Ser Ala Ile Glu Asp Leu Leu Phe Asp Lys Val Val Thr Ser
                965                 970                 975

GGC TTA GGT ACA GTT GAT GAA GAT TAT AAA CGT TGT ACA GGT GGT TAT 3072
Gly Leu Gly Thr Val Asp Glu Asp Tyr Lys Arg Cys Thr Gly Gly Tyr
            980                 985                 990

GAC ATA GCT GAC TTA GTG TGT GCA CAA TAT TAC AAT GGC ATC ATG GTG 3120
Asp Ile Ala Asp Leu Val Cys Ala Gln Tyr Tyr Asn Gly Ile Met Val
        995                 1000                1005

CTA CCT GGT GTA GCT AAT GAT GAC AAG ATG GCT ATG TAC ACT GCA TCT 3168
Leu Pro Gly Val Ala Asn Asp Asp Lys Met Ala Met Tyr Thr Ala Ser
    1010                1015                1020

CTT GCA GGT GGT ATA ACA TTA GGT TCA CTT GGT GGT GGC GCA GTG TCT 3216
Leu Ala Gly Gly Ile Thr Leu Gly Ser Leu Gly Gly Gly Ala Val Ser
1025                1030                1035                1040

ATA CCT TTT GCA ATA GCA GTT CAA GCC AGA CTT AAT TAT GTT GCT CTA 3264
Ile Pro Phe Ala Ile Ala Val Gln Ala Arg Leu Asn Tyr Val Ala Leu
                1045                1050                1055

CAA ACT GAT GTA TTG AAC AAG AAC CAG CAG ATC CTG GCT AAT GCT TTC 3312
Gln Thr Asp Val Leu Asn Leu Asn Gln Gln Ile Leu Ala Asn Ala Phe
            1060                1065                1070

AAT CAA GCT ATT GGT AAC ATT ACA CAG GCA TTT GGT AAG GTT AAT GAT 3360
Asn Gln Ala Ile Gly Asn Ile Thr Gln Ala Phe Gly Lys Val Asn Asp
        1075                1080                1085

GCT ATA CAT CAA ACG TCA CAA GGT CTT GCT ACT GTT GCT AAA GTA TTG 3408
Ala Ile His Gln Thr Ser Gln Gly Leu Ala Thr Val Ala Lys Val Leu
    1090                1095                1100

GCA AAA GTG CAA GAT GTT GTT AAC ACA CAA GGG CAA GCT TTA AGC CAC 3456
Ala Lys Val Gln Asp Val Val Asn Thr Gln Gly Gln Ala Leu Ser His
1105 1110 115 1120

CTA ACA TTA CAA TTG CAA AAT AAT TTC CAA GCC ATT AGT AGT TCC ATT 3504
Leu Thr Leu Gln Leu Gln Asn Asn Phe Gln Ala Ile Ser Ser Ser Ile
                1125                1130                1135

AGT GAC ATT TAT AAC AGG CTT GAT GAA TTG AGT GCT GAT GCA CAA GTT 3552
Ser Asp Ile Tyr Asn Arg Leu Asp Glu Leu Ser Ala Asp Ala Gln Val
            1140                1145                1150

GAC AGG CTG ATT ACA GGA AGA CTT ACA GCA CTT AAT GCA TTT GTG TCT 3600
Asp Arg Leu Ile Thr Gly Arg Leu Thr Ala Leu Asn Ala Phe Val Ser
        1155                1160                1165

CAG ACT TTA ACC AGA CAA GCA GAG GTT AGG GCT AGC AGA CAG CTT GCT 3648
Gln Thr Leu Thr Arg Gln Ala Glu Val Arg Ala Ser Arg Gln Leu Ala
    1170                1175                1180

AAA GAC AAG GTA AAT GAA TGC GTC AGG TCT CAA TCT CAG AGA TTT GGA 3696
Lys Asp Lys Val Asn Glu Cys Val Arg Ser Gln Ser Gln Arg Phe Gly
1185                1190                1195                1200

TTC TGT GGT AAT GGT ACA CAT TTA TTT TCA CTT GCA AAT GCA GCA CCA 3744
Phe Cys Gly Asn Gly Thr His Leu Phe Ser Leu Ala Asn Ala Ala Pro
                1205                1210                1215

AAT GGC ATG ATC TTC TTT CAC ACA GTG CTA TTA CCA ACA GCT TAT GAA 3792
Asn Gly Met Ile Phe Phe His Thr Val Leu Leu Pro Thr Ala Tyr Glu
            1220                1225                1230

ACC GTG ACA GCC TGG TCA GGT ATT TGT GCA TCA GAT GGC GAT CGT ACT 3840
Thr Val Thr Ala Trp Ser Gly Ile Cys Ala Ser Asp Gly Asp Arg Thr
        1235                1240                1245

TTT GGA CTT GTT GTT AAG GAT GTC CAG TTG ACG CTG TTT CGC AAT CTA 3888
Phe Gly Leu Val Val Lys Asp Val Gln Leu Thr Leu Phe Arg Asn Leu
    1250                1255                1260

GAT GAC AAA TTC TAT TTG ACT CCC AGA ACT ATG TAT CAG CCT ATA GTT 3936
Asp Asp Lys Phe Tyr Leu Thr Pro Arg Thr Met Tyr Gln Pro Ile Val
1265                1270                1275                1280

GCA ACT AGT TCT GAT TTT GTT CAA ATT GAA GGA TGT GAT GTG TTG TTT 3984
Ala Thr Ser Ser Asp Phe Val Gln Ile Glu Gly Cys Asp Val Leu Phe
                1285                1290                1295

GTT AAT GCA ACT GTA ATT GAC TTG CCT AGT ATT ATA CCT GAC TAT ATT 4032
Val Asn Ala Thr Val Ile Asp Leu Pro Ser Ile Ile Pro Asp Tyr Ile
            1300                1305                1310

GAT ATT AAT CAA ACT GTT CAG GAC ATA TTA GAA AAT TTC AGA CCA AAT 4080
Asp Ile Asn Gln Thr Val Gln Asp Ile Leu Glu Asn Phe Arg Pro Asn
        1315                1320                1325

TGG ACT GTA CCT GAG TTG CCA CTT GAC ATT TTC AAT GCA ACC TAC TTA 4128
Trp Thr Val Pro Glu Leu Pro Leu Asp Ile Phe Asn Ala Thr Tyr Leu
    1330                1335                1340

AAC CTG ACT GGT GAA ATT AAT GAC TTA GAA TTT AGG TCA GAA AAG TTA 4176
Asn Leu Thr Gly Glu Ile Asn Asp Leu Glu Phe Arg Ser Glu Lys Leu
1345                1350                1355                1360

CAT AAC ACC ACA GTA GAA CTT GCT ATT CTC ATT GAT AAT ATT AAT AAC 4224
His Asn Thr Thr Val Glu Leu Ala Ile Leu Ile Asp Asn Ile Asn Asn
                1365                1370                1375

ACA TTA GTC AAT CTT GAA TGG CTC AAT AGA ATT GAA ACT TAT GTA AAA 4272
Thr Leu Val Asn Leu Glu Trp Leu Asn Arg Ile Glu Thr Tyr Val Lys
            1380                1385                1390

TGG CCT TGG TAT GTG TGG CTA CTA ATT GGA TTA GTA GTA ATA TTC TGC 4320
Trp Pro Trp Tyr Val Trp Leu Leu Ile Gly Leu Val Val Ile Phe Cys
        1395                1400                1405

ATA CCC ATA TTG CTA TTT TGT TGT TGT AGT ACT GGT TGT TGT GGA TGT 4368
Ile Pro Ile Leu Leu Phe Cys Cys Cys Ser Thr Gly Cys Cys Gly Cys
    1410                1415                1420

ATT GGG TGT TTA GGA AGC TGT TGT CAT TCC ATA TGT AGT AGA AGG CAA 4416
Ile Gly Cys Leu Gly Ser Cys Cys His Ser Ile Cys Ser Arg Arg Gln
1425                1430                1435                1440

TTT GAA AGT TAT GAA CCA ATT GAA AAA GTT CAT GTT CAC TGA ATTC 4462
Phe Glu Ser Tyr Glu Pro Ile Glu Lys Val His Val His ***
1445 1450 1453

## Claims

1. Polynucleotide comprising a nucleotide sequence coding for at least part of the E₂ protein of Canine Corona Virus (CCV).

2. Polynucleotide selected from:
a. DNA having at least part of the nucleotide sequence represented in Sequence ID No: 1;
b. polynucleotides which hybridize to any of the foregoing DNA and which codes for a polypeptide having immunogenic properties of the E₂ protein of CCV
c. polynucleotides which are degenerate as a result of the genetic code to the DNA as defined in (a) or the polynucleotides as defined in (b) and which code for a polypeptide having immunogenic properties of the E₂ protein of CCV.

3. Recombinant polynucleotide coding for the expressing of at least part of the E₂ protein of CCV, characterized in that it contains a suitable vector and a polynucleotide according to claim 1 or 2 inserted therein.

4. Polypeptide having at least part of the amino acid sequence of the E₂ protein of CCV.

5. Polypeptide having at least part of the amino acid sequence represented in Sequence ID No: 1.

6. Vaccine for combating CCV infection containing an effective amount of a recombinant polynucleotide according to claim 3 or a polypeptide according to claim 4 or 5.

7. Use of a polypeptide according to claim 4 or 5 in combating CCV infections.
